(19) Europäisches Patentamt
European Patent Office
Office européen des brevets

(11) Numéro de publication: **0 003 147**
**B1**

(12) **FASCICULE DE BREVET EUROPEEN**

(45) Date de publication du fascicule de brevet: **30.12.81**

(51) Int. Cl.[3]: **C 01 B 7/19**, C 07 C 17/38

(21) Numéro de dépôt: **79200010.1**

(22) Date de dépôt: **09.01.79**

(54) **Procédé pour la séparation du fluorure d'hydrogène de ses mélanges avec le 1-chloro-1,1-difluoréthane.**

(30) Priorité: **13.01.78 FR 7801309**

(43) Date de publication de la demande:
**25.07.79 Bulletin 79/15**

(45) Mention de la délivrance du brevet:
**30.12.81 Bulletin 81/52**

(84) Etats Contractants Désignés:
**BE CH DE FR GB IT LU NL SE**

(56) Documents cités:
**US - A - 2 640 086**

(73) Titulaire: **SOLVAY & Cie (Société Anonyme)**
**Rue du Prince Albert, 33**
**B-1050 Bruxelles (BE)**

(72) Inventeur: **Lorquet, Robert**
**Avenue du Touquet 23**
**B-1330 Rixensart (BE)**

(74) Mandataire: **Eischen, Roland**
**Solvay & Cie Département de la Propriété Industrielle Rue de Ransbeek 310**
**B-1120 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 003 147 B1

# Procédé pour la séparation du fluorure d'hydrogène de ses mélanges avec le 1-chloro-1,1-difluoréthane

L'invention concerne un procédé pour la séparation du fluorure d'hydrogène de ses mélanges avec le 1-chloro-1,1-difluoréthane. Elle s'applique notamment à la séparation du fluorure d'hydrogène non transformé contenu dans les mélanges résultant de la fabrication du 1-chloro-1,1-difluoréthane par hydrofluoration d'hydrocarbures chlorés. Elle s'applique tout particulièrement à la séparation du fluorure d'hydrogène contenu dans les mélanges à base de 1-chloro-1,1-difluoréthane résultant de l'hydrofluoration du chlorure de vinylidène ou du 1,1,1-trichloréthane.

La plupart des procédés de fabrication du 1-chloro-1,1-difluoréthane par hydrofluoration donnent des mélanges complexes contenant notamment du 1-chloro-1,1-difluoréthane et du fluorure d'hydrogène non transformé. Il en est ainsi du procédé de fabrication du 1-chloro-1,1-difluoréthane par hydrofluoration du chlorure de vinylidène ou du 1,1,1-trichloréthane.

Pour séparer du 1-chloro-1,1-difluoréthane pur de ces mélanges, il est courant de les soumettre à un lavage avec une phase aqueuse (brevet britannique 1 323 234 déposé le 27.7.1971 au nom de Daikin Kogyo Co Ltd) pouvant contenir un acide ou une base (demande de brevet japonais 47/39 086 déposée le 31.8.1964 au nom de Kureha Chemical Ind. Co Ltd).

Ces procédés connus présentent l'inconvénient grave de récupérer le fluorure d'hydrogène sous forme de solutions aqueuses de fluorures inorganiques ou d'acide fluorhydrique.

En effet, pour obtenir un taux de transformation suffisant de l'hydrocarbure chloré de départ, on est amené à mettre en oeuvre à l'hydrofluoration un excès important de fluorure d'hydrogène qu'il est hautement souhaitable, pour des raisons économiques, de récupérer sous forme anhydre afin de pouvoir le recycler à la réaction.

Dans le but de récupérer le fluorure d'hydrogène sous forme anhydre, on ne peut recourir à une simple distillation, comme cela a déjà été proposé pour séparer les mélanges de fluorure d'hydrogène avec le 1,1-dichloro-1-fluoréthane (Br. Etats-Unis 2 894 044 déposé le 16.2.1956 par Monsanto Chem. Co.), car le fluorure d'hydrogène et le 1-chloro-1,1-difluoréthane forment un azéotrope. Par ailleurs, la séparation par décantation ne peut être envisagée en pratique vu les solubilités mutuelles élevées du fluorure d'hydrogène et du 1-chloro-1,1-difluoréthane.

D'ailleurs, on n'a jamais proposé de séparer par décantation le fluorure d'hydrogène et le 1-chloro-1,1-difluoréthane. Pareille séparation n'a été exploitée jadis que pour séparer le fluorure d'hydrogène du chlorodifluorméthane en leur ajoutant du trichlorométhane (brevet des Etats-Unis 2 640 086 déposé le 15 décembre 1951 et cédé à E.I. du Pont de Nemours and Co).

La Demanderesse a maintenant trouvé un procédé qui ne présente plus les inconvénients mentionnés ci-dessus des procédés connus.

La présente invention concerne donc un procédé pour la séparation du fluorure d'hydrogène de ses mélanges avec le 1-chloro-1,1-difluoréthane selon lequel on soumet le mélange liquide à une séparation par décantation de manière à obtenir une phase organique liquide enrichie en 1-chloro-1,1-difluoréthane et une phase inorganique liquide enrichie en fluorure d'hydrogène et selon lequel on ajoute au mélange un liquide contenant un tiers solvant constitué d'au moins 50% en poids en 1,1-dichloro-1-fluoréthane avant de le soumettre à la décantation.

Les tiers solvants mis en oeuvre dans le procédé selon l'invention, peuvent contenir, outre le 1,1-dichloro-1-fluoroéthane, du chlorure de vinylidène ou du 1,1,1-trichloréthane.

Les liquides ajoutés selon l'invention peuvent être constitués de produits purs ou de mélanges. Ils sont en général constitués à raison d'au moins 20% en poids de tiers solvants, d'excellents résultats étant obtenus lorsqu'ils sont constitués à raison d'au moins 50% en poids de tiers solvants. Il est bien entendu que les meilleurs résultats sont obtenus lorsque le liquide ajouté est formé exclusivement de ces tiers solvants; cependant pour des raisons de simplification du procédé, comme il apparaît plus loin, on utilise le plus souvent des liquides contenant de 50 à 100% en poids de tiers solvants.

Lorsque le mélange à traiter provient de l'hydrofluoration du chlorure de vinylidène, on préfère mettre en oeuvre des liquides contenant du 1,1-dichloro-1-fluoréthane et du chlorure de vinylidène comme seuls tiers solvants. De même lorsque le mélange à traiter provient de l'hydrofluoration du 1,1,1-trichloréthane, on préfère mettre en oeuvre des liquides contenant du 1,1-dichloro-1-fluoréthane et du 1,1,1-trichloréthane comme seuls tiers solvants. Dans tous les cas, les meilleurs résultats sont obtenus lorsque les tiers solvants sont constitués d'au moins 50% et de préférence d'au moins 80% en poids de 1,1-dichloro-1-fluoréthane par rapport à la totalité des tiers solvants.

En plus des tiers solvants, les liquides ajoutés selon l'invention peuvent également contenir sans inconvénient du fluorure d'hydrogène et du 1-chloro-1,1-difluoréthane. Ces composés sont en général présents à raison de moins de 80% et de préférence moins de 50% en poids. C'est ainsi qu'on peut utiliser comme liquides des mélanges réactionnels provenant de l'hydrofluoration du chlorure de vinylidène ou du 1,1,1-trichloréthane, éventuellement débarassés du chlorure d'hydrogène sous-produit, et contenant des quantités importantes de produit intermédiaire (1,1-dichloro-1-fluoréthane), des quantités variables de réactif organique de départ (chlorure de vinylidène ou 1,1,1-trichloréthane) et des quantités moins importantes de produit (1-chloro-1,1-difluoréthane) et de fluorure d'hydrogène. Bien entendu, on peut aussi utiliser des produits épurés et des mélanges provenant de divers traitements de séparation auxquels les mélanges réactionnels peuvent être soumis.

La quantité de liquide à ajouter peut varier dans d'assez larges limites. En général, le liquide est mis en oeuvre dans des proportions telles que les tiers solvants (1,1-dichloro-1-fluoréthane, chlorure de vinylidène et 1,1,1-trichloréthane) soient présents à raison de 0,01 à 5 et de préférence de 0,05 à 2,5 mole de tiers solvant par mole de 1-chloro-1,1-difluoréthane présent dans le mélange complexe soumis à la décantation qui comprend donc le mélange à traiter et le liquide ajouté. Des quantités comprises entre 0,1 et 1 mole par mole conviennent particulièrement bien. Pour certaines applications, où il n'est pas indispensable de réduire de façon très importante la teneur en fluorure d'hydrogène et en 1-chloro-1,1-difluoréthane respectivement l'un dans l'autre, on peut mettre en oeuvre des quantités moins importantes de tiers solvants. On peut également mettre en oeuvre des quantités plus importantes de tiers solvant, mais, d'un point de vue économique cette façon d'opérer présente moins d'intérêt, du fait que l'on doit soumettre la phase organique liquide à une distillation ultérieure pour séparer le 1-chloro-1,1-difluoréthane. De plus l'avantage qui découle de la diminution de la solubilité du fluorure d'hydrogène dans la phase organique, lors de la mise en oeuvre de quantités très importantes de tiers solvant, est au moins partiellement compensé par le fait que la quantité totale de fluorure d'hydrogène dissous dans la phase organique est bien entendu proportionnelle au volume de cette phase.

Le procédé selon l'invention convient pour traiter des mélanges contenant une quantité prépondérante de fluorure d'hydrogène et de 1-chloro-1,1-difluoréthane en toutes proportions relatives. Il convient particulièrement bien pour des mélanges contenant de 0,1 à 50 mole de fluorure d'hydrogène par mole de 1-chloro-1,1-difluoréthane. D'excellents résultats ont été obtenus en traitant suivant le procédé de l'invention, des mélanges contenant de 0,2 à 10 mole de fluorure d'hydrogène par mole de 1-chloro-1,1-difluoréthane.

Les mélanges de fluorure d'hydrogène et de 1-chloro-1,1-difluoréthane que l'on peut traiter peuvent également contenir d'autres composés. Parmi ceux-ci, le 1,1-dichloro-1-fluoréthane, le chlorure de vinylidène et le 1,1,1-trichloréthane ne sont évidemment pas gênants, tout au contraire, et il est avantageux de tenir compte de leur présence éventuelle pour évaluer la quantité de tiers solvant à ajouter. Le procédé selon l'invention est généralement applicable aux mélanges contenant au moins 50% en poids de fluorure d'hydrogène et de 1-chloro-1,1-difluoréthane. On préfère l'appliquer aux mélanges contenant au moins 80% en poids de ces composés, les meilleurs résultats étant obtenus lorsqu'ils ne contiennent que ces composés. Parmi les autres composés que le mélange peut contenir, le chlorure d'hydrogène et les autres hydrocarbures halogénés comprenant de 1 à 6 atomes de carbone figurent le plus souvent. Cependant, lorsque le mélange de départ provient de l'hydrofluoration du chlorure de vinylidène ou du 1,1,1-trichloréthane, il est avantageux de le débarrasser, avant de le traiter selon l'invention, au moins partiellement du chlorure d'hydrogène sous-produit, par exemple par distillation.

Selon un mode de réalisation avantageux de l'invention, on traite un mélange de 1-chloro-1,1-difluoréthane et de fluorure d'hydrogène correspondant à la composition azéotropique.

Les mélanges de 1-chloro-1,1-difluoréthane et de fluorure d'hydrogène à traiter selon l'invention sont obtenus de toute manière connue en elle-même. En général ils proviennent de l'hydrofluoration du chlorure de vinylidène ou du 1,1,1-trichloréthane. Dans ce cas il est avantageux de débarrasser la composition ainsi obtenue, avant de la traiter selon l'invention, au moins partiellement du chlorure d'hydrogène sous-produit, par exemple par distillation. Cette séparation du chlorure d'hydrogène peut avantageusement être suivie d'une séparation, au moins partielle d'au moins un des constituants de la composition choisi parmi les réactifs non transformés, les sous-produits de la réaction et éventuellement le constituant choisi parmi le fluorure d'hydrogène et le 1-chloro-1,1-difluoréthane, qui est en excès par rapport à la composition azéotropique de manière à obtenir le mélange à traiter suivant l'invention.

Le liquide peut être ajouté sous forme gazeuse ou liquide au mélange à traiter, ce dernier pouvant être lui-même gazeux ou liquide. Après cette addition, ou maintient le mélange complexe ainsi obtenu à une température et une pression choisies de manière à maintenir ou amener le mélange complexe sous forme liquide en vue de séparer par décantation les deux phases liquides, organique et inorganique. En général, on utilise des températures ne dépassant pas 110°C et de préférence 90°C. Des températures comprises entre —50 et +80°C sont utilisées avec succès. La pression peut être légèrement inférieure, égale, ou supérieure à la pression atmosphérique. En général on utilise des pressions allant de 0,8 à 30 bar et de préférence de 1 à 20 bar.

La séparation par décantation peut être réalisée en continu ou en discontinu. Elle peut être réalisée selon diverses techniques connues en elles-mêmes telles que décantation par gravité ou par action de la force centrifuge, ou passage au travers de membranes poreuses sélectivement mouillées par l'une ou l'autre phase. Divers types d'appareils connus en eux-mêmes peuvent être utilisés à cette fin. On peut ainsi utiliser des décanteurs florentins, des décanteurs centrifuges, des filtres séparateurs à membranes ou des séparateurs électriques. La séparation par décantation peut être facilitée par une opération préalable de coalescence des gouttelettes dans des appareils connus en eux-mêmes tels, que des matelas ou des cartouches en matériaux fibreux mouillables de préférence par la phase dispersée.

La séparation par décantation selon l'invention peut avantageusement être combinée avec d'autres opérations de séparation telles que des distillations. C'est ainsi que le fluorure d'hydrogène présent dans la phase inorganique liquide peut être efficacement séparé du 1-chloro-1,1-difluoréthane au moyen d'une distillation opérée dans des conditions qui conviennent pour la distillation d'un mélange de liquides peu solubles l'un dans l'autre et formant un azéotrope à minimum. De même, le 1-chloro-1,1-difluoréthane présent dans la phase organique liquide peut être séparé au moyen d'une distillation semblable permettant de recueillir en pied une phase organique exempte de fluorure d'hydrogène que l'on peut soumettre à une seconde distillation pour séparer le 1-chloro-1,1-difluoréthane.

Selon un mode de réalisation préféré de l'invention, on opère la séparation par décantation dans un condenseur-décanteur commun, où l'on condense le produit de tête de deux distillations, qui constitue le mélange à traiter selon l'invention, où l'on ajoute le liquide contenant un tiers solvant, où l'on prélève la phase organique liquide pour le reflux d'une distillation et la phase inorganique liquide pour le reflux de l'autre. Le condenseur-décanteur peut être constitué d'un appareil unique où les opérations de condensation et de décantation ont lieu simultanément ou de deux appareils distincts successifs où ont lieu respectivement chacune des deux opérations.

Un tel procédé de séparation comprend avantageusement les opérations suivantes:

(a) première distillation de manière à obtenir en tête de colonne un mélange contenant essentiellement du fluorure d'hydrogène et du 1-chloro-1,1-difluoréthane dans des proportions voisines de celles correspondant à l'azéotrope, et en pied de colonne une première fraction liquide contenant le constituant qui, dans l'ensemble des produits admis à la première distillation est en excès par rapport à la composition azéotropique.

(b) séparation par décantation en une phase inorganique liquide enrichie en fluorure d'hydrogène et une phase organique liquide enrichie en 1-chloro-1,1-difluoréthane après condensation du mélange recueilli en tête de colonne et addition du liquide contenant un tiers solvant.

(c) recyclage pour le reflux de la première distillation de la phase liquide obtenue à la séparation par décantation qui contient le constituant qui, dans la première distillation est en excès par rapport à la composition azéotropique.

(d) deuxième distillation de l'autre phase liquide obtenue à la séparation par décantation, de manière à obtenir en tête de colonne un mélange contenant essentiellement du fluorure d'hydrogène et du 1-chloro-1,1-difluoréthane dans des proportions voisines de celles correspondant à l'azéotrope qui comme le produit de tête de la première distillation est soumis à la condensation et à la séparation par décantation et en pied de colonne une seconde fraction liquide contenant le constituant qui dans l'ensemble des produits admis à la première distillation est en défaut par rapport à la composition azéotropique.

Par ensemble des produits admis à la première distillation, la Demanderesse entend désigner la phase liquide obtenue à la décantation qui est recyclée pour le reflux de la première distillation et les autres produits éventuellement admis à cette distillation.

La composition de l'azéotrope du fluorure d'hydrogène avec le l'chloro-1,1-difluoréthane, sous une pression de 5 bars est de 38 à 48% en mole de fluorure d'hydrogène d'après la demande de brevet japonais 49/125 286 déposée le 6.4.1973 au nom de Daikin Kogyo Co Ltd. Si l'ensemble des produits admis à la première distillation contient moins de fluorure d'hydrogène que la composition azéotropique, c'est-à-dire moins d'environ 40% en mole de fluorure d'hydrogène, la première fraction liquide recueillie en pied de la première distillation contient du 1-chloro-1,1-difluoréthane et est quasiment exempte de fluorure d'hydrogène et la seconde fraction liquide recueillie en pied de la deuxième distillation contient du fluorure d'hydrogène et est quasiment exempte de 1-chloro-1,1-difluoréthane. Dans le cas contraire, c'est-à-dire lorsque l'ensemble des produits admis à la première distillation contient plus d'environ 40% en mole de fluorure d'hydrogène, la première fraction liquide recueillie au pied de la première distillation contient du fluorure d'hydrogène et est quasiment exempte de 1-chloro-1,1-difluoréthane et la seconde fraction liquide recueillie au pied de la deuxième distillation contient du 1-chloro-1,1-difluoréthane et est quasiment exempte de fluorure d'hydrogène.

Le procédé de l'invention peut être utilisé avantageusement en combinaison avec des distillations pour la séparation du 1-chloro-1,1-difluoréthane du fluorure d'hydrogène obtenus par hydrofluoration du chlorure de vinylidène et du 1,1,1-trichloréthane. Le produit de la réaction peut après séparation du chlorure d'hydrogène être soumis directement à la première distillation. Dans ce cas, le liquide contenant un tiers solvant peut être avantageusement mis en oeuvre directement à la séparation par décantation. Le produit de la réaction peut aussi, s'il contient une quantité suffisante de tiers solvants, être mis en oeuvre directement à la séparation par décantation. Dans ce cas, les deux distillations sont alimentées uniquement par reflux des deux phases liquides séparées. Dans les deux cas, le mélange à traiter selon l'invention est le mélange ayant une composition voisine de la composition azéotropique recueilli en tête des distillations.

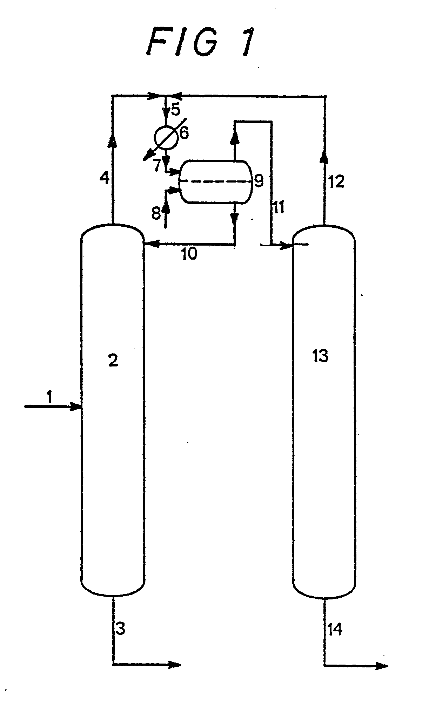

Le procédé suivant l'invention peut avantageusement être réalisé dans des installations telles que celles schématisées aux figures 1 à 4 des dessins annexés.

La description de ces installations concerne la séparation de compositions telles que l'ensemble des produits admis à la première distillation contienne un défaut de fluorure d'hydrogène par rapport à la composition azéotropique. Il est bien évident que les mêmes installations peuvent être utilisées pour séparer des compositions pour lesquelles l'ensemble des produits admis à la première distillation contient un excès de fluorure d'hydrogène par rapport à la composition azéotropique. Dans ce cas les points de prélèvement des deux produits et les tuyauteries de reflux sont inversés.

La figure 1 représente un ensemble de colonnes à distillation azéotropique ayant un condenseur commun.

On introduit par la voie 1 dans la colonne à distiller 2 une composition contenant du fluorure d'hydrogène et du 1-chloro-1,1-difluoréthane. On recueille en 4 en continu un mélange de fluorure d'hydrogène et de 1-chloro-1,1-difluoréthane à traiter selon l'invention ayant une composition voisine de la composition azéotropique. Le mélange est envoyé par la voie 5 au condenseur 6 et ensuite par la voie 7 au décanteur 9. Le décanteur 9 est alimenté par la voie 8 par le liquide contenant un tiers solvant conformément au procédé de l'invention. La phase organique obtenue au décanteur est recyclée par la voie 10 à la colonne à distiller 2. En pied de la colonne 2 on recueille par la voie 3 le 1-chloro-1,1-difluoréthane en mélange avec le tiers solvant. La phase inorganique obtenue au décanteur est envoyée par la voie 11 dans une seconde colonne à distiller 13 au pied de laquelle on recueille par la voie 14 le fluorure d'hydrogène. En tête de la colonne 13 on recueille par la voie 12 un mélange de fluorure d'hydrogène et de 1-chloro-1,1-difluoréthane à traiter selon l'invention ayant une composition voisine de la composition azéotropique que l'on recycle au condenseur 6 par la voie 5.

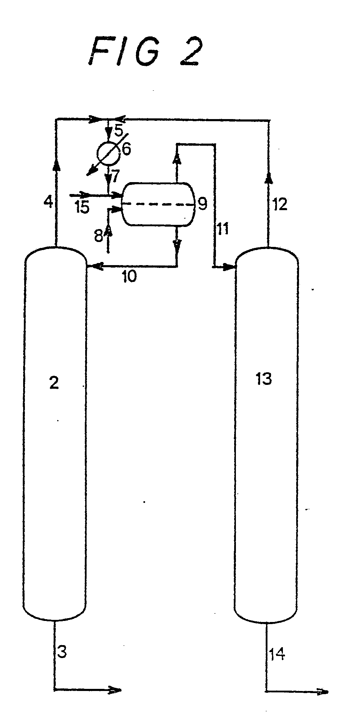

La figure 2 représente une variante de l'installation schématisée à la figure 1 dans laquelle la composition de fluorure d'hydrogène et de 1-chloro-1,1-difluoréthane est introduite par la voie 15 et mélangée avec la composition azéotropique de fluorure d'hydrogène et de 1-chloro-1,1-difluoréthane recueillie en 7, le mélange étant envoyé au décanteur 9 pour y être traité selon l'invention.

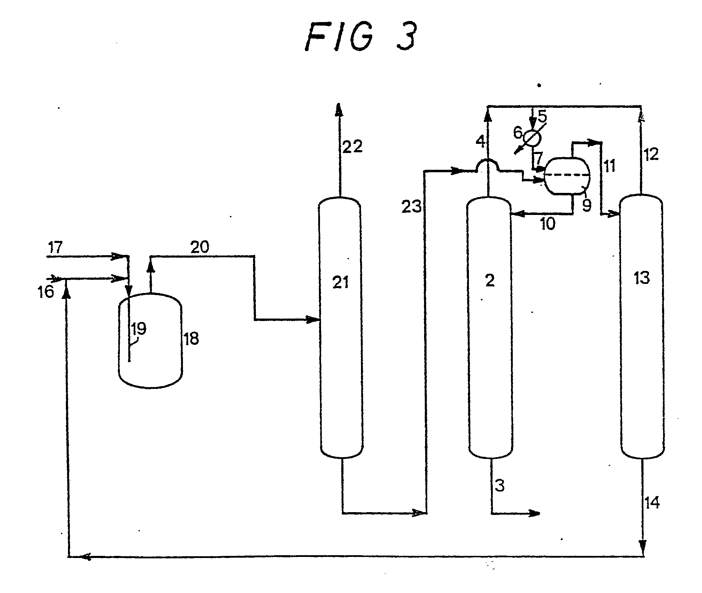

La figure 3 représente une installation complète combinant celle schématisée à la figure 2 avec celle de fabrication du 1-chloro-1,1-difluoréthane par hydrofluoration aussi bien du chlorure de vinylidène que du 1,1,1-trichloréthane.

L'hydrocarbure chloré (voie 17) et le fluorure dhydrogène (voie 16) sont introduits par la voie 19 dans le réacteur d'hydrofluoration 18. On soutire en continu par la voie 20 la composition obtenue que l'on envoie dans la colonne à distiller 21. En tête de la colonne 21 on sépare le chlorure d'hydrogène de cette composition. Les autres constituants, soit notamment le fluorure d'hydrogène, le 1-chloro-1,1-difluoréthane, ainsi que le 1,1-dichloro-1-fluoréthane sous-produit et éventuellement l'hydrocarbure chloré non transformé, sont envoyés directement par la voie 23 dans le décanteur 9 où ils exercent la fonction de liquide ajouté. On recueille en 14 le fluorure d'hydrogène que l'on recycle. Le mélange d'hydrocarbures halogénés recueilli en 3 est soumis à des distillations subséquentes de manière à en séparer les divers constituants. L'hydrocarbure chloré non transformé et le 1,1-dichloro-1-fluoréthane peuvent être recyclés au réacteur ou à la décantation 9.

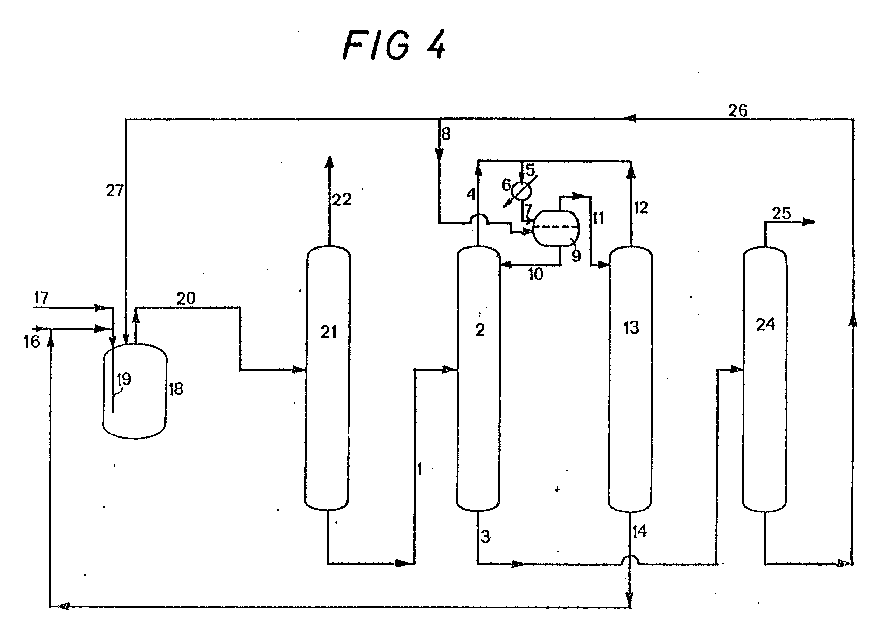

La figure 4 représente un autre type d'installation complète dans laquelle la composition recueillie au pied de la colonne de séparation du chlorure d'hydrogène 21, est envoyée par la voie 1 dans la colonne à distiller 2. On recueille en 4 en continu le mélange de composition voisine de la composition azéotropique. Au pied de la colonne 2 on soutire, par la voie 3, le 1-chloro-1,1-difluoréthane en mélange avec le liquide et on l'envoie dans la colonne 24 dont on soutire en tête par la voie 25 le 1-chloro-1,1-difluoréthane et en pied par la voie 26 le liquide que l'on envoie partiellement par la voie 8 au décanteur 9. L'autre partie du liquide est recyclée par la voie 27 au réacteur.

Le procédé faisant l'objet de la présente invention est particulièrement intéressant car il permet de récupérer le fluorure d'hydrogène sous forme anhydre de ses mélanges avec le 1-chloro-1,1-difluoréthane.

Grâce à l'introduction du tiers solvant, la séparation des phases organique et inorganique est quasi immédiate alors que, en l'absence de ce dernier, elle dure extrêmement longtemps. De plus le procédé selon l'invention permet de diminuer fortement la teneur résiduelle en fluorure d'hydrogène de la phase organique et la teneur résiduelle en 1-chloro-1,1-difluoréthane de la phase inorganique. Ainsi, en utilisant des installations telles que celles schématisées aux figures 1 et 2, on peut abaisser la concentration du fluorure d'hydrogène et du 1-chloro-1,1-difluoréthane l'un dans l'autre à moins de 1% de manière à obtenir du fluorure d'hydrogène quasiment exempt de 1-chloro-1,1-difluoréthane et du 1-chloro-1,1-difluoréthane quasiment exempt de fluorure d'hydrogène. En outre, il permet une séparation simple et efficace du mélange réactionnel en tous ses constituants sans introduire d'additifs extérieurs au procédé.

Enfin, étant donné que les tiers solvants selon l'invention ont une densité supérieure à celle du fluorure d'hydrogène, leur introduction dans le mélange à séparer facilite la décantation.

Le fluorure d'hydrogène, que l'invention permet de séparer, peut être recyclé ou utilisé dans d'autres fabrications.

Le 1-chloro-1,1-difluoréthane est couramment utilisé pour la synthèse de monomères fluorés tels que le fluorure de vinylidène qui est lui-même utilisé pour la fabrication de polymères à haute résistance aux agents chimiques.

Afin d'illustrer l'invention, sans en limiter la portée, on donne ci-après des exemples de réalisation.

Exemples 1 et 2

Deux essais de séparation du fluorure d'hydrogène de ses mélanges avec le 1-chloro-1,1-difluoréthane sont réalisés en présence de 1,1-dichloro-1-fluoréthane (essai 1) et, à titre de comparaison, en l'absence de 1,1-dichloro-1-fluoréthane (essai 2R). La température est de −20°C et la pression de 1 bar.

Les résultats obtenus sont présentés au Tableau I.

TABLEAU I

| | COMPOSITION | |
|---|---|---|
| | ESSAI 1 | ESSAI 2R |
| Produits mis en oeuvre | | |
| 1-chloro-1,1-difluoréthane | 3,1 mole | 3,7 mole |
| fluorure d'hydrogène | 3,3 mole | 2,5 mole |
| 1,1-dichloro-1-fluoréthane | 1,0 mole | − |
| Produits décantés a l'équilibre | | |
| − phase organique | | |
| fluorure d'hydrogène | 7% molaires | 18% molaires |
| 1-chloro-1,1-difluoréthane | 70% molaires | 82% molaires |
| 1,1-dichloro-1-fluoréthane | 23% molaires | − |
| − phase inorganique | | |
| fluorure d'hydrogène | 90% molaires | 77% molaires |
| 1-chloro-1,1-difluoréthane | 8% molaires | 23% molaires |
| 1,1-dichloro-1-fluoréthane | 2% molaires | − |

L'examen des résultats présentés au Tableau I montre que grâce à l'introduction de 1,1-dichloro-1-fluoréthane dans le mélange à séparer on parvient à réduire sensiblement la teneur en composés organiques du fluorure d'hydrogène et la teneur en fluorure d'hydrogène des composés organiques.

Exemple 3

Cet exemple est réalisé afin de mettre en évidence l'amélioration de la séparation du fluorure d'hydrogène et du 1-chloro-1,1-difluoréthane grâce à l'introduction de 1,1-dichloro-1-fluoréthane dans le condenseur-décanteur unique d'un système de distillation azéotropique à deux colonnes.

Les essais sont réalisés dans une installation analogue à celle représentée à la figure 1.

On introduit par heure dans la colonne 2 par la voie 1 un mélange contenant 50 moles de fluorure d'hydrogène et 16 moles de 1-chloro-1,1-difluoréthane sous une pression de 9 bars. La température en tête de la colonne 2 est de 48°C. La phase gazeuse est condensée, et envoyée au décanteur 9 qui est alimenté par 22 moles par heure de 1,1-dichloro-1-fluoréthane.

La phase organique recueillie dans le décanteur est renvoyée à la colonne 2 et la phase inorganique est distillée dans la colonne 13 dont on soutire en tête, à une température de 48,5°C, une phase gazeuse contenant du fluorure d'hydrogène et du 1-chloro-1,1-difluoréthane que l'on condense en 6 et que l'on envoie au décanteur 9.

La phase organique recueillie en 3 au pied de la colonne 2 contient le 1-chloro-1,1-difluoréthane et le 1,1-dichloro-1-fluoréthane et moins de 1% de fluorure d'hydrogène. Le fluorure d'hydrogène recueilli en 14 au pied de la colonne 13 contient moins de 5% de 1-chloro-1,1-difluoréthane.

**Revendications**

1. Procédé pour la séparation du fluorure d'hydrogène de ses mélanges avec le 1-chloro-1,1-difluoréthane selon lequel on soumet le mélange liquide à une séparation par décantation de manière à obtenir une phase organique liquide enrichie en 1-chloro-1,1-difluoréthane et une phase inorganique liquide enrichie en fluorure d'hydrogène caractérisé en ce que l'on ajoute au mélange un liquide contenant un tiers solvant constitué d'au moins 50% en poids de 1,1-dichloro-1-fluoroéthane avant de le soumettre à la décantation.

2. Procédé suivant la revendication 1 caractérisé en ce que le tiers solvant est constitué d'au moins 80% en poids de 1,1-dichloro-1-fluoroéthane.

3. Procédé suivant la revendication 1 ou 2, caractérisé en ce que le liquide ajouté contient moins de 50% en poids de 1-chloro-1,1-difluoroéthane et de fluorure d'hydrogène.

4. Procédé suivant l'une quelconque des revendications 1 à 3, caractérisé en ce que le liquide ajouté est mis en oeuvre à raison de 0,05 à 2,5 mole de tiers solvant par mole de 1-chloro-1,1-difluoréthane.

5. Procédé suivant l'une quelconque des revendications 1 à 4, caractérisé en ce que la séparation est effectuée à une température inférieure à 110°C.

6. Procédé suivant l'une quelconque des revendications 1 à 5, caractérisé en ce que la séparation est opérée à une pression allant de 0,8 à 30 bar.

7. Procédé suivant l'une quelconque des revendications 1 à 6 caractérisé en ce que le liquide ajouté est un mélange réctionnel provenant de l'hydrofluoration du chlorure de vinylidène.

8. Procédé suivant l'une quelconque des revendications 1 à 7, caractérisé en ce que le liquide ajouté est un mélange réactionnel provenant de l'hydrofluoration du 1,1,1-trichloréthane.

9. Procédé suivant l'une quelconque des revendications 1 à 8, caractérisé en ce que le mélange de fluorure d'hydrogène et de 1-chloro-1,1-difluoréthane soumis à la séparation contient au moins 80% en poids de fluorure d'hydrogène et de 1-chloro-1,1-difluoréthane.

10. Procédé suivant l'une quelconque des revendications 1 à 9, caractérisé en ce que le mélange de fluorure d'hydrogène et de 1-chloro-1,1-difluoréthane soumis à la séparation a une composition voisine de la composition azéotropique.

11. Procédé suivant l'une quelconque des revendications 1 à 10, caractérisé en ce que la séparation est opérée dans un condenseur-décanteur où l'on condense le produit de tête de deux distillations, où l'on ajoute le liquide, et où l'on prélève la phase organique liquide pour le reflux d'une distillation et la phase inorganique liquide pour le reflux de l'autre.

**Claims**

1. Process for the separation of hydrogen fluoride from its mixtures with 1-chloro-1,1-difluoroethane, in accordance with which the liquid mixture is subjected to separation by decantation so as to obtain a liquid organic phase enriched in 1-chloro-1,1-difluoroethane and a liquid inorganic phase enriched in hydrogen fluoride, characterised in that a liquid containing an auxiliary solvent, consisting of at least 50% by weight of 1,1-dichloro-1-fluoroethane, is added to the mixture before subjecting it to decantation.

2. Process according to Claim 1, characterised in that the auxiliary solvent consists of at least 80% by weight of 1,1-dichloro-1-fluoroethane.

3. Process according to Claim 1 or 2, characterised in that the added liquid contains less than 50% by weight of 1-chloro-1,1-difluoroethane and of hydrogen fluoride.

4. Process according to any one of Claims 1 to 3, characterised in that the added liquid is used in an amount of 0.05 to 2.5 mols of auxiliary solvent per mol of 1-chloro-1,1-difluoroethane.

5. Process according to any one of Claims 1 to 4, characterised in that the separation is carried out at a temperature below 110°C.

6. Process according to any one of Claims 1 to 5, characterised in that the separation is carried out at a pressure ranging from 0.8 to 30 bars.

7. Process according to any one of Claims 1 to 6, characterised in that the added liquid is a reaction mixture originating from the hydrofluorination of vinylidene chloride.

8. Process according to any one of Claims 1 to 7, characterised in that the added liquid is a reaction mixture originating from the hydrofluorination of 1,1,1-trichloroethane.

9. Process according to any one of Claims 1 to 8, characterised in that the mixture of hydrogen fluoride and 1-chloro-1,1-difluoroethane subjected to the separation contains at least 80% by weight of hydrogen fluoride and of 1-chloro-1,1-difluoroethane.

10. Process according to any one of Claims 1 to 9, characterised in that the mixture of hydrogen fluoride and 1-chloro-1,1-difluoroethane subjected to the separation has a composition similar to the azeotropic composition.

11. Process according to any one of claims 1 to 10, characterised in that the separation is carried out in a condenser/decanter in which the first fraction of two distillations is condensed, to which the liquid is added, and from which the liquid organic phase is removed for the reflux of one distillation and the liquid inorganic phase is removed for reflux of the other distillation.

**Patentansprüche**

1. Verfahren zur Trennung von Fluorwasserstoff aus seinen Mischungen mit 1-Chlor-1,1-difluoräthan, wobei man die flüssige Mischung einer Trennung durch Dekantieren derart unterwirft, daß man eine flüssige, mit 1-Chlor-1,1-Difluoräthan angereicherte organische Phase und eine flüssige mit Fluorwasserstoff angereicherte anorganische Phase erhält, dadurch gekennzeichnet, daß man zu der Mischung eine Flüssigkeit zugibt, die ein drittes Lösungsmittel enthält bestehend aus mindestens 50 Gew.-% 1,1-Dichlor-1-fluoräthan, bevor man die Mischung der Dekantierung unterwirft.

2. Verfahren gemäß Anspruch 1, dadurch gekennzeichnet, daß das dritte Lösungsmittel aus mindestens 80 Gew.-% 1,1-Dichlor-1-fluoräthan besteht.

3. Verfahren gemäß Anspruch 1 oder 2, dadurch gekennzeichnet, daß die zugesetzte Flüssigkeit weniger als 50 Gew.-% 1-Chlor-1,1-difluoräthan und Fluorwasserstoff enthält.

4. Verfahren gemäß einem der Ansprüche 1 bis 3, dadurch gekennzeichnet, daß die zugesetzte Flüssigkeit eingesetzt wird in einem Verhältnis von 0,05 bis 2,5 Mol der dritten Flüssigkeit je Mol 1-Chlor-1,1-difluoräthan.

5. Verfahren gemäß einem der Ansprüche 1 bis 4, dadurch gekennzeichnet, daß die Trennung bei einer Temperatur unterhalb von 110°C durchgeführt wird.

6. Verfahren gemäß einem der Ansprüche 1 bis 5, dadurch gekennzeichnet, daß die Trennung bei einem Druck von 0,8 bis 30 bar durchgeführt wird.

7. Verfahren gemäß einem der Ansprüche 1 bis 6, dadurch gekennzeichnet, daß die zugesetzte Flüssigkeit eine Reaktionsmischung aus der Hydrofluorierung von Vinylidenchlorid ist.

8. Verfahren gemäß einem der Ansprüche 1 bis 7, dadurch gekennzeichnet, daß die zugesetzte Flüssigkeit eine Reaktionsmischung aus der Hydrofluorierung von 1,1,1-Trichloräthan ist.

9. Verfahren gemäß einem der Ansprüche 1 bis 8, dadurch gekennzeichnet, daß die der Trennung unterworfene Mischung von Fluorwasserstoff und 1-Chlor-1,1-difluoräthan mindestens 80 Gew.-% Fluorwasserstoff und 1-Chlor-1,1-difluoräthan enthält.

10. Verfahren gemäß einem der Ansprüche 1 bis 9, dadurch gekennzeichnet, daß die der Trennung unterworfene Mischung von Fluorwasserstoff und 1-Chlor-1,1-difluoräthan eine Zusammensetzung in der Nähe der azeotropen Mischung besitzt.

11. Verfahren gemäß einem der Anspruch 1 bis 10, dadurch gekennzeichnet, daß die Trennung in einer Kondensator-Dekantiervorrichtung durchgeführt wird, wo man das Kopfprodukt von zwei Destillationen kondensiert, die Flüssigkeit zusetzt und die flüssige organische Phase abnimmt zum Refluxieren einer Destillation und die flüssige anorganische Phase zum Refluxieren der anderen.

FIG 1

5
6
7
8
9
4
12
11
10
2
13
1
3
14

*FIG 2*

5
6
7
4
15
9
12
8
11
10
2
13
3
14

FIG 3

22
4
5
6
7
11
12
23
9
10
17
20
16
21
2
13
19
18
3
14

FIG 4